# EUROPEAN PATENT APPLICATION

(11) **EP 4 767 975 A2**
(43) Date of publication of application: **01.07.2026**
(21) Application number: 26150060.7
(22) Date of filing: 02.01.2026
(51) Int. Cl.: A61B 18/14, A61B 17/00, A61B 18/12, A61B 18/16

(54) **TECHNOLOGIES FOR SEGMENTED ELECTRODES FOR ENERGY-BASED SURGICAL INSTRUMENTS**

(30) Priority: 31.12.2024 US 202463740963 P; 30.09.2025 US 202519345060
(71) Applicant: Cilag GmbH International, 6300 Zug (CH)
(72) Inventor: SHELTON, IV, Frederick E., Cincinnati, 45242 (US); ARONHALT, Jacqueline, Cincinnati, 45242 (US); HARRIS, Jason L., Cincinnati, 45242 (US); LUCAS, Guion, Cincinnati, 45242 (US); MESSERLY, Jeffrey D., Cincinnati, 45242 (US); BREHM, Tyler, Cincinnati, 45242 (US)
(74) Representative: Carpmaels & Ransford LLP

(57) **Abstract**

A system includes an energy-based surgical instrument with an end effector having one or more jaw clamps. The end effector may include a segmented electrode with a distal portion and a proximal portion coupled to one jaw clamp and a return electrode coupled to the other jaw clamp. The distal and proximal portions may be separately or cooperatively energized with radio frequency (RF) energy. A pressure varying mechanism may selectively limit or increase pressure at the distal portion of the electrode. The end effector may include a jaw clamp with a tissue pad positioned within a channel. The end effector may include a conformal or erodible electrode that extends to the distal tip of the jaw assembly. Other embodiments are described and claimed.

## Description

### CROSS-REFERENCE TO RELATED APPLICATION

This application claims the benefit of and priority to U.S. Patent Application No. 63/740,963, entitled "TECHNOLOGIES FOR TISSUE PADS AND ELECTRODES FOR ENERGY-BASED SURGICAL INSTRUMENTS," which was filed on December 31, 2024, and which is incorporated herein by reference in its entirety.

### TECHNICAL FIELD

The present disclosure relates generally to energy-based surgical instruments and, more particularly, to harmonic and/or electrosurgical surgical instruments.

### BACKGROUND

Energy-based surgical instruments are finding increasingly widespread applications in surgical procedures by virtue of their unique performance characteristics. Depending upon specific device configurations and operational parameters, energy-based surgical instruments can provide both transection of tissue and hemostasis of the tissue by coagulation, which may reduce or otherwise minimize patient trauma. Depending on the particular application, energy-based surgical instruments may utilize different surgical technologies including, for example, ultrasonic and/or electro-surgical (e.g., radio frequency (RF)) technologies.

A typical ultrasonic surgical instrument may include a handpiece containing an ultrasonic transducer and an elongated shaft assembly having a distally mounted end effector to effect the cutting and sealing of tissue. For example, the end effector may include a jaw assembly having an ultrasonic blade and a clamp arm, which may include a non-stick tissue pad or similar bed to receive the ultrasonic blade. In some cases, the elongated shaft assembly may be permanently affixed to the handpiece. In other cases, the elongated shaft assembly may be detachable from the handpiece, as in the case of a disposable shaft assembly or a shaft assembly that is interchangeable between different handpieces. In use, the end effector transmits ultrasonic energy to tissue brought into contact with the ultrasonic blade of the end effector to realize the cutting and sealing action. Such ultrasonic surgical devices may be configured for open surgical use, laparoscopic, and/or endoscopic surgical procedures including robotic-assisted procedures.

Ultrasonic energy cuts and coagulates tissue using temperatures lower than those used in electro-surgical procedures. Vibrating at high frequencies (e.g., 55,500 times per second), the ultrasonic blade denatures protein in the tissue to form a sticky coagulum. Pressure exerted on tissue by the ultrasonic blade surface collapses blood vessels and allows the coagulum to form a hemostatic seal. A surgeon can control the cutting speed and coagulation by the force applied to the tissue by the end effector, the time over which the force is applied, and the selected excursion level of the end effector.

In electro-surgical instruments, one or more electrodes are incorporated into the end effector and configured to apply therapeutic electrical current to the patient's tissue to create a hemostatic seal. In electro-surgical instruments that do not include a harmonic mode (i.e., do not include a harmonic blade), the end effector may be embodied as two clamp arms or jaws. In such embodiments, the electro-surgical instrument may include a separate mechanical knife or blade for cutting the tissue after the creation of the hemostatic seal, which may be incorporated into the elongated shaft attached to the end effector. In bi-polar embodiments, an active electrode may be attached to one of the clamp arms of the end effector and configured to introduce an electrical current into the tissue, which is received by a return electrode attached to the other clamp arm of the end effector (or as the blade itself in embodiments including a harmonic mode). Conversely, in mono-polar embodiments, the return electrode (e.g., a "grounding pad") may be separate from the electro-surgical instrument and located on a different part of the body of th1e patient. In some embodiments, the electro-surgical instrument may also be configured to apply a sub-therapeutic electrical current to the patient's tissue, which may be used for sensing purposes (e.g., measuring tissue impedance).

Electro-surgery forms hemostatic seals by generating heat in the tissue via the introduced electrical energy, which is embodied as radio frequency ("RF") energy. The particular frequency employed can vary based on the intended use of the electro-surgical instrument within the range of about 100 kHz to 1 MHz, although higher frequencies can be employed in some embodiments. Additionally, sub-therapeutic frequencies may be used in some situations for purposes other than hemostatic sealing, such as performing various electrical measurements on the tissue.

It should be appreciated that some energy-based surgical instruments may employ dual or multi-modal technologies for the transection and/or hemostasis of patient tissue. For example, in some cases, an energy-based surgical instrument may include both ultrasonic and electro-surgical capabilities (e.g., by utilizing the ultrasonic blade as an electrode for the electro-surgery mode), which increases the surgical options provided by the surgical instrument to the surgeon.

### SUMMARY

According to one aspect of the disclosure, an energy-based surgical instrument includes an end effector comprising a first jaw clamp and a second jaw clamp, a first electrode coupled to the first jaw clamp, and a return electrode coupled to the second jaw clamp. The first electrode includes a distal portion and a proximal portion. The distal portion and the proximal portion are independently energizable to deliver radio frequency (RF) energy through tissue between the first jaw clamp and the second jaw clamp to the return electrode.

In some embodiments, the proximal portion is configured to deliver a coagulation energy, and the distal portion is configured to separately or cooperatively deliver the coagulation energy or a cutting energy. In some embodiments, the cutting energy is higher than the coagulation energy. In some embodiments, the distal portion is configured to separately deliver the cutting energy when tissue is only within the distal portion of the first jaw clamp. In some embodiments, the distal portion is configured cooperatively deliver the coagulation energy or the cutting energy when tissue is within the distal portion and the proximal portion of the first jaw clamp.

In some embodiments, the end effector further includes a pressure varying mechanism that selectively provides limited pressure or concentrated pressure on the distal portion of the first electrode. In some embodiments, the end effector further includes a rotational mechanism that rotates a hump under the first electrode or the second electrode, wherein when the hump is rotated, pressure on the distal portion of the first electrode is increased.

In some embodiments, the return electrode includes a distal portion. The distal portion includes a spring deflectable portion that is spring biased toward the first electrode and is deflectable away from the first electrode. In some embodiments, the spring deflectable portion includes a deflection stop limit that limits deflection of the return electrode away from the first electrode. In some embodiments, when the first jaw clamp and the second jaw clamp are closed with tissue between the distal portion and the proximal portion of the first electrode and the return electrode, the spring deflectable portion does not reach the deflection stop limit, and pressure applied at the distal portion is limited. When the first jaw clamp and the second jaw clamp are closed with tissue between the distal portion of the first electrode and the return electrode and without tissue between the proximal portion of the first electrode and the return electrode, the spring deflectable portion reaches the deflection stop limit, and increased pressure is applied at the distal portion. In some embodiments, the surgical instrument further includes a protrusion extending from the distal portion of the return electrode toward the distal portion of the first electrode. In some embodiments, the deflection stop limit is movable between a first position in which deflection of the return electrode is inhibited and a second position in which deflection of the return electrode is not inhibited.

According to another aspect, an energy-based surgical instrument includes an end effector including a jaw clamp, a tissue pad coupled to the jaw clamp, and an electrode. The jaw clamp extends from a proximal end to a distal tip. The tissue pad extends from the proximal end to the distal tip. The tissue pad includes a non-conductive top surface having proximal pad geometry. The electrode is coupled to the top surface of the tissue pad and extends from the proximal end to the distal tip. The electrode covers a first portion of the tissue pad and does not cover a second portion of the tissue pad. The electrode includes a distal feature that extends to the distal tip of the jaw clamp, and the distal feature has a different retention shape or geometry than the proximal pad geometry.

In some embodiments, the electrode comprises a conformal or erodible RF electrode for use with an ultrasonic blade. In some embodiments, the distal feature of the electrode interacts electrically with tissue before a proximal portion of the electrode interacts electrically with the tissue. In some embodiments, the tissue pad comprises polytetrafluoroethylene (PTFE).

According to another aspect, a method for controlling an energy-based surgical instrument includes clamping tissue between a first jaw clamp and a second jaw clamp of an end effector of the energy-based surgical instrument, wherein the energy-based surgical instrument comprises a first electrode coupled to the first jaw clamp, the first electrode comprising a distal portion and a proximal portion, and wherein the energy-based surgical instrument further comprises a return electrode coupled to the second jaw clamp; independently energizing the distal portion to deliver radio frequency (RF) energy through the tissue between the first jaw clamp and the second jaw clamp to the return electrode when the tissue is only within the distal portion of the first jaw clamp; and cooperatively energizing the distal portion and the proximal portion to deliver RF energy through the tissue between the first jaw clamp and the second jaw clamp to the return electrode when the tissue is within the distal portion and the proximal portion of the first jaw clamp.

In some embodiments, independently energizing the distal portion comprises delivering a cutting energy; and cooperatively energizing the distal portion and the proximal portion comprises delivering a coagulation energy. In some embodiments, the cutting energy is higher than the coagulation energy.

In some embodiments, the method further includes providing concentrated pressure on the distal portion of the first electrode with a pressure varying mechanism when independently energizing the distal portion. In some embodiments, the method further includes providing limited pressure on the distal portion of the first electrode with the pressure varying mechanism when cooperatively energizing the distal portion and the proximal portion.

### BRIEF DESCRIPTION OF THE DRAWINGS

The detailed description particularly refers to the following figures, in which:
FIG. 1 is a simplified diagram of an embodiment of a system for performing an energy-based surgical procedure;
FIG. 2 is a perspective view of an embodiment of an energy-based surgical instrument of the system of FIG. 1;
FIG. 3 is a side elevation view of a jaw assembly of an end effector of the surgical instrument of FIG. 2 including an ultrasonic blade and in an open state;
FIG. 4 is a side elevation view of the jaw assembly of the end effector of the surgical instrument of FIG. 2 including an ultrasonic blade and in a closed state;
FIG. 5A is a perspective view of another embodiment of the end effector of the surgical instrument of FIG. 2 including an electrode on a lower jaw clamp of the jaw assembly;
FIG. 5B is a perspective view of another embodiment of the end effector of the surgical instrument of FIG. 2 including two jaw clamps, each having an electrode attached thereto;
FIG. 6 is an exploded view of the surgical instrument of FIG. 2;
FIG. 7 is a block diagram of a control circuit of the surgical instrument of FIG. 2;
FIG. 8 is a perspective view of another embodiment of the end effector of the surgical instrument of FIG. 2 including a segmented cutting electrode;
FIG. 9 is a simplified flow diagram of at least one method for controlling the surgical instrument of FIG. 8; and
FIG. 10 is a top view of another embodiment of a jaw clamp assembly of an end effector of the surgical instrument of FIG. 2 including a compliant electrode.

### DETAILED DESCRIPTION OF THE DRAWINGS

While the concepts of the present disclosure are susceptible to various modifications and alternative forms, specific illustrative embodiments thereof have been shown by way of example in the drawings and will herein be described in detail. It should be understood, however, that there is no intent to limit the concepts of the present disclosure to the particular forms disclosed, but on the contrary, the intention is to cover all modifications, equivalents, and alternatives falling within the spirit and scope of the invention as defined by the appended claims.

Terms representing anatomical references, such as anterior, posterior, medial, lateral, superior, inferior, distal, proximal, et cetera, may be used throughout the specification in reference to the surgical instruments described herein as well as in reference to the patient's natural anatomy. Such terms have well-understood meanings in both the study of anatomy and the field of surgery. Use of such anatomical reference terms in the written description and claims is intended to be consistent with their well-understood meanings unless noted otherwise.

References in the specification to "one embodiment,""an embodiment," "an illustrative embodiment," etc., indicate that the embodiment described may include a particular feature, structure, or characteristic, but every embodiment may or may not necessarily include that particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to effect such feature, structure, or characteristic in connection with other embodiments whether or not explicitly described. Additionally, it should be appreciated that items included in a list in the form of "at least one A, B, and C" can mean (A); (B); (C); (A and B); (A and C); (B and C); or (A, B, and C). Similarly, items listed in the form of "at least one of A, B, or C" can mean (A); (B); (C); (A and B); (A and C); (B and C); or (A, B, and C).

The disclosed embodiments may be implemented, in some cases, in hardware, firmware, software, or any combination thereof. The disclosed embodiments may also be implemented as instructions carried by or stored on a transitory or non-transitory machine-readable (e.g., computer-readable) storage medium, which may be read and executed by one or more processors. A machine-readable storage medium may be embodied as any storage device, mechanism, or other physical structure for storing or transmitting information in a form readable by a machine (e.g., a volatile or non-volatile memory, a media disc, or other media device).

In the drawings, some structural or method features may be shown in specific arrangements and/or orderings. However, it should be appreciated that such specific arrangements and/or orderings may not be required. Rather, in some embodiments, such features may be arranged in a different manner and/or order than shown in the illustrative figures. Additionally, the inclusion of a structural or method feature in a particular figure is not meant to imply that such feature is required in all embodiments and, in some embodiments, may not be included or may be combined with other features.

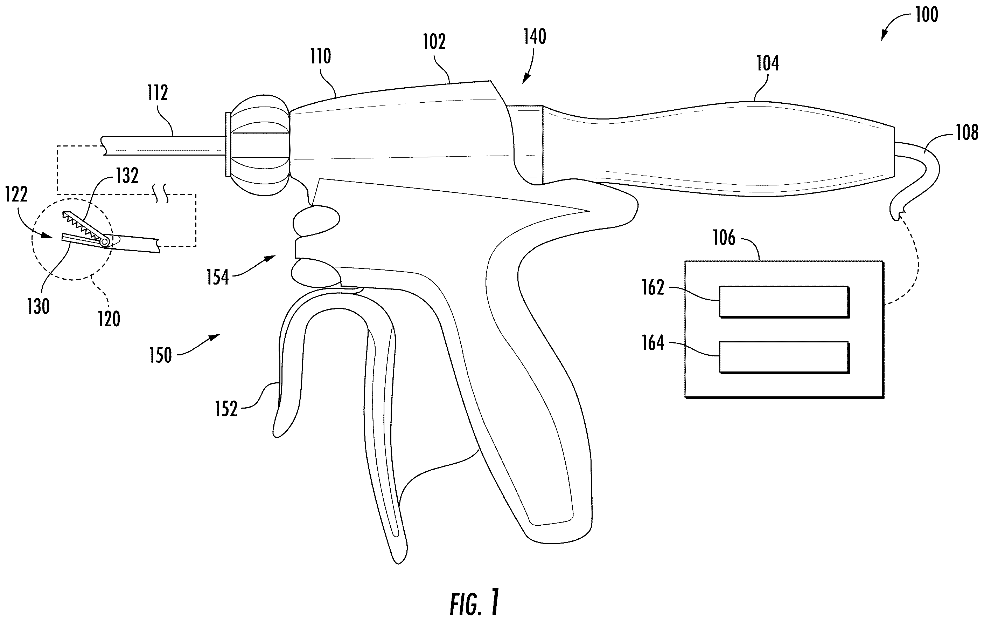

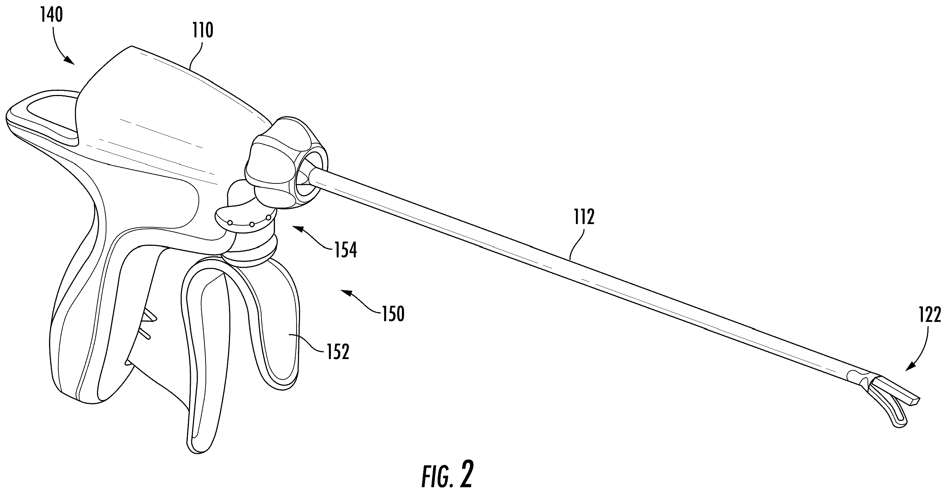

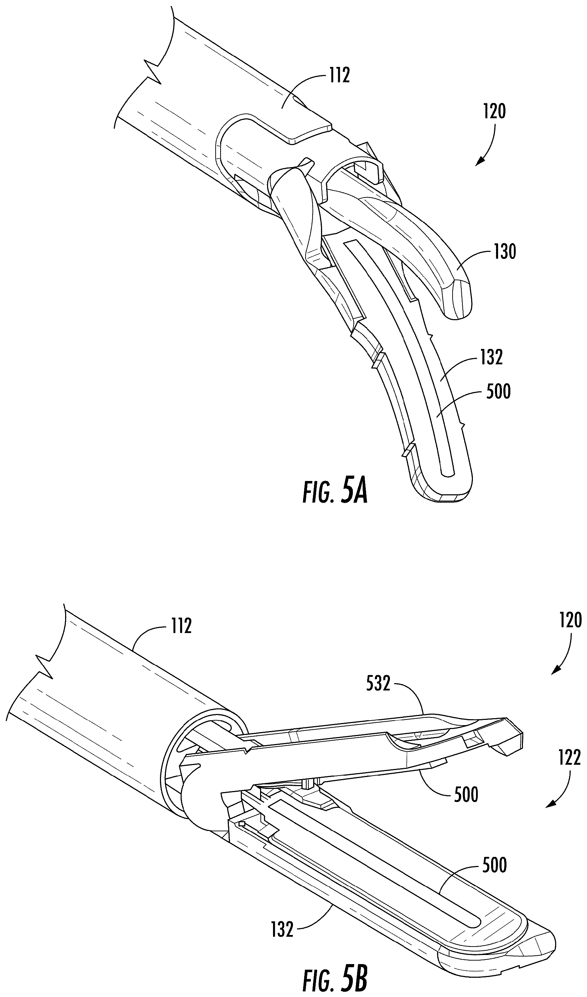

Referring now to FIGS. 1 and 2, in an illustrative embodiment, a system 100 for performing an energy-based surgical procedure includes a surgical instrument 102, a transducer 104, and a generator 106. The surgical instrument 102 is illustratively embodied as an ultrasonic surgical instrument, but may be embodied as an electro-surgical surgical instrument or a multi-modal, ultrasonic/elector-surgical surgical instrument in other embodiments. In use, the surgical instrument 102 is usable to perform various surgical procedures including laparoscopic, endoscopic, or traditional open surgical procedures. In doing so, a surgeon may selectively activate an ultrasonic mode (and/or an electro-surgical/RF mode) of the surgical instrument 102. In the ultrasonic mode, the generator 106 drives the transducer 104 to cause an ultrasonic blade 130 of a jaw assembly 122 of an end effector 120 of the surgical instrument 102 to vibrate at a reference frequency, which facilitates the contemporaneous cutting and hemostatic sealing of patient tissue. Additionally or alternatively, in some embodiments, the surgeon may selectively activate an electro-surgical mode of the surgical instrument 102 to deliver an amount of therapeutic RF energy to the patient tissue to effect hemostatic sealing. In such embodiments, the blade 130 may be embodied as an ultrasonic blade 130 or as a mechanical blade designed to cut tissue using mechanical force (e.g., in those embodiments not employing ultrasonic technologies). Furthermore, in some embodiments, the surgical instrument 102 may be configured with only an electro-surgical/RF mode and, in such embodiments, the jaw assembly 122 of the end effector 120 may not include the ultrasonic blade 130 as discussed in more detail below in regard to FIG. 5B.

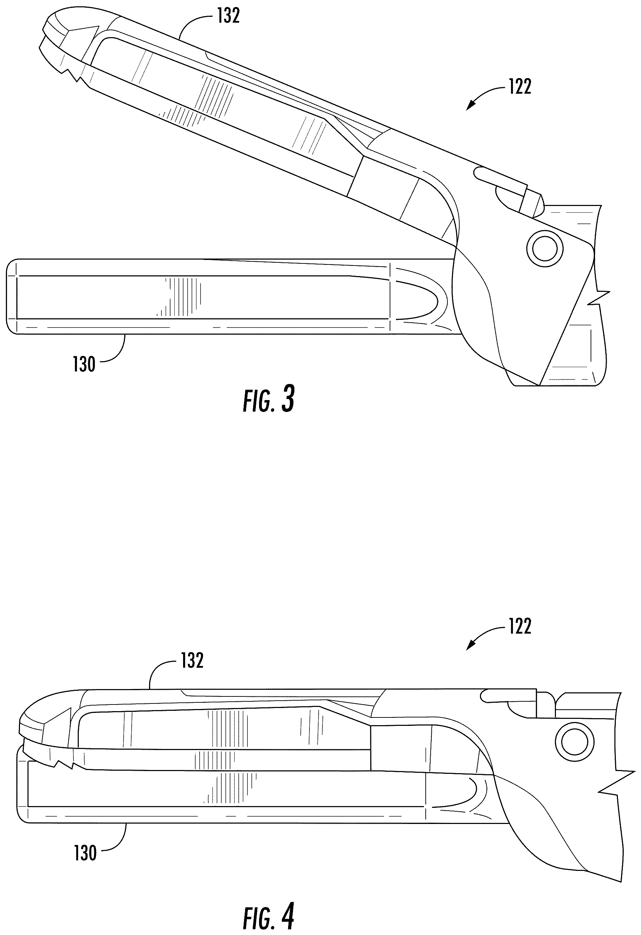

The surgical instrument 102 is illustratively embodied as ultrasonic surgical shears but may be embodied as other types of surgical instruments having an ultrasonic mode and/or electro-surgical mode in other embodiments. In the illustrative embodiment, the surgical instrument 102 includes a handle assembly 110 and an elongated shaft assembly 112, which extends distally away from the handle assembly 110 and may be removably attached to the handle assembly 110 in some embodiments. The elongated shaft assembly 112 includes the end effector 120 located at a distal end opposite the handle assembly 110. The end effector 120 includes the jaw assembly 122, which illustratively includes the ultrasonic blade 130 and a corresponding jaw clamp 132 (but may include two jaw clamps in those embodiments having only an electro-surgical/RF mode). As shown in FIGS. 3 and 4, the jaw assembly 122 is movable between an open state (FIG. 3) in which the jaw clamp 132 is positioned away from the ultrasonic blade 130 and a closed state (FIG. 4) in which the jaw clamp 132 is positioned near or otherwise contacts the ultrasonic blade 130. Actuation of the jaw assembly 122 from the open state to the closed state allows for the grasping, cutting, and coagulation of vessels and/or tissue by the jaw assembly 122. It should be appreciated that the open state may correspond to a degree of openness that is less than a fully opened position of the jaw assembly 122 and the closed state may correspond to a degree of closeness that is less than a fully closed position. That is, the closed state may, for example correspond to a minimal distance between the distal ends of the jaw clamp 132 and the ultrasonic blade 130 and the open state may correspond to a maximum distance between the distal ends of the jaw clamp 132 and the ultrasonic blade 130. However, in other embodiments, the open state may correspond to a fully opened position of the jaw assembly 122 and the closed state may correspond to a fully closed position of the jaw assembly 122.

In those embodiments in which the surgical instrument 102 includes both a ultrasonic mode and an electro-surgical/RF mode, the end effector 120 may include one or more RF electrodes 500 incorporated into the jaw clamp 132 as shown in FIG. 5A. Although the illustrative end effector 120 includes only a single electrode 500 in the embodiment of FIG. 5A, it should be appreciated that the end effector 120 may include additional electrodes 500 in other embodiments (e.g., multiple pads of electrodes 500). The electrode(s) 500 may be embodied as an active electrode configured to the RF energy or as a return electrode configured to "sink" an applied RF energy. In those embodiments utilizing bi-polar RF implementation, the ultrasonic blade 130 may embody the active or return electrode, with the electrode 500 embodying the other active or return electrode. Alternatively, other active or return electrodes may be incorporated on the ultrasonic blade 130 or in another part of the jaw assembly 122 of the end effector 120. In mono-polar implementation, the RF electrode(s) 500 may be embodied as an active electrode, and a return electrode may be attached to a portion of the patient's body.

In those embodiments in which the surgical instrument 102 includes only an electro-surgical/RF mode, the jaw assembly 122 of the end effector 120 includes a jaw clamp 532 in place of the ultrasonic blade 130 as shown in FIG. 5B. In such embodiments, an electrode 500 may be attached to or otherwise incorporated into each jaw clamp 132, 532 and be embodied as an active or a return electrode to facilitate the application of RF energy to tissue captured between the jaw clamps 132, 532. In such embodiments, the surgical instrument 102 may include a knife incorporated into the elongated shaft assembly 112 that is configured to eject outwardly to cut the patient's tissue after sealing of the tissue by the RF energy.

Referring back to FIGS. 1 and 2, in those embodiments including ultrasonic capabilities, the handle assembly 110 includes a receptacle 140 configured to receive the transducer 104 to facilitate connection of the transducer 104 to the handle assembly 110 and the elongated shaft assembly 112. The handle assembly 110 also includes a trigger assembly 150, which includes a primary trigger 152 and a switch assembly 154. The primary trigger 152 is operable by the surgeon to move the jaw assembly 122 of the end effector 120 between the open and closed states. The switch assembly 154 includes one or more buttons, which are selectable by the surgeon to activate (and configure, in some embodiments) the ultrasonic mode and/or the electro-surgical mode of the surgical instrument 102.

The transducer 104 is illustratively connected to the generator 106 by a cable assembly 108. As discussed above, the generator 106 is configured to drive the transducer 104 at a reference or resonant frequency to thereby cause the ultrasonic blade 130 to vibrate. For example, in an illustrative embodiment, the generator 106 may supply an electrical signal to the transducer 104 to cause the ultrasonic blade 130 of the jaw assembly 122 to vibrate longitudinally in the range of, for example, approximately 20 kHz to 250 kHz. In particular embodiments, for example, the ultrasonic blade 130 may vibrate in the range of about 54 kHz to 56 kHz (e.g., at about 55.5 kHz). In other embodiments, the ultrasonic blade 130 may vibrate at other frequencies including, for example, about 31 kHz or about 80 kHz. The excursion of the vibrations at the ultrasonic blade 130 can be controlled by, for example, controlling the amplitude of the electrical signal applied to the transducer 104 by the generator 106. The generator 106 may be activated so that electrical energy may be continuously or intermittently supplied to the transducer 104. The generator 106 also has a power line (not shown) for insertion in an electro-surgical unit or conventional electrical outlet. Additionally or alternatively, the generator 106 may be powered by a direct current (DC) source, such as a battery.

In some embodiments, the generator 106 may be configured to operate in different modes. In such embodiments, the generator 106 may include an ultrasonic generator module 162 for controlling an ultrasonic mode, an electro-surgical/Radio Frequency (RF) generator module 164 for controlling an electro-surgical mode, and/or other generator modules (e.g., a heat generator module) for controlling other operation modes. The various modes of the generator 106 may be operated independently of each other in some embodiments. For example, the generator 106 may activate the ultrasonic mode of the ultrasonic generator module 162 to apply ultrasonic energy to the jaw assembly 122 and subsequently, either therapeutic or sub-therapeutic RF energy may be applied to the jaw assembly 122 by the electro-surgical generator module 164. Alternatively, the activation modes of the generator 106 may be operated simultaneously or contemporaneously with each other.

In the electro-surgical mode, the electro-surgical generator module 164 is configured to generate RF energy at a frequency in the range of about 100 kilohertz (100 kHz) to about 1 megahertz (1 MHz). The generated RF energy is supplied to the patient's tissue via the electrodes 500 of the end effector 120 as described above in regard to FIG. 5. In some embodiments, the electro-surgical generator module 164 may also be configured to selectively provide the RF energy at sub-therapeutic levels to perform various electrical measurements of the patient's tissue. For example, the electro-surgical generator module 164 may be configured to measure an impedance of the patient's tissue using the electrodes 500 and a suitable RF energy level.

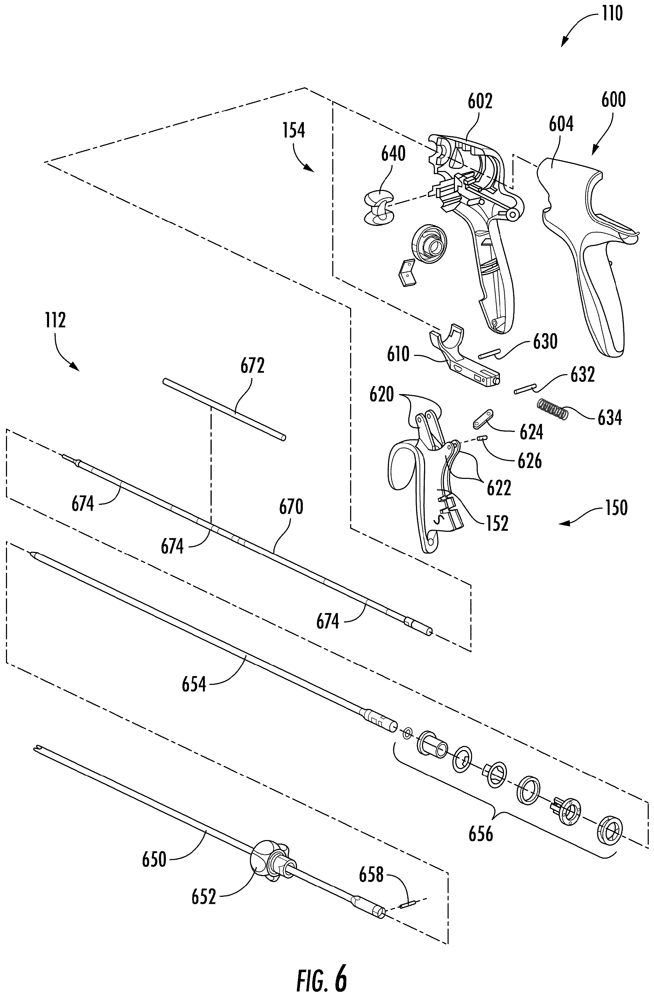

Referring now to FIG. 6, as discussed above, the illustrative surgical instrument 102 includes the handle assembly 110 and the elongated shaft assembly 112, which extends distally away from the handle assembly 110. The handle assembly 110 includes a housing 600, which includes a right half-housing 602 and a left half-housing 604. The half-housings 602, 604 are configured to mate with each other to form the housing 600. To facilitate such mating, each of the half-housings 602, 604 may include various interfaces sized to mechanically align and engage one another to form the housing 600 and enclose the internal working components of the surgical instrument 102.

The primary trigger 152 of the trigger assembly 150 is coupled to a linkage mechanism to translate the rotational motion of the primary trigger 152 to axial motion of a yoke 610, which in turn is configured to move the jaw assembly 122 of the end effector 120 between the open and closed states via the elongated shaft assembly 112. The primary trigger 152 includes a first set of flanges 620 having openings formed therein to receive a first yoke pin 630, which extends through the yoke 610. The primary trigger 152 also includes a second set of flanges 622 configured to receive a first end of a link 624. A trigger pin 626 is received in openings formed in the first end of the link 624 and the second set of flanges 622. The trigger pin 626 forms a trigger pivot point for the primary trigger 152. A second end of the link 624, opposite the first end, is received in a slot formed in a proximal end of the yoke 610 and retained therein by a second yoke pin 632. As the primary trigger 152 is rotated about the pivot point formed from the trigger pin 626, the yoke 610 translates horizontally. A spring 634 is used to bias the yoke forward such that the jaw assembly 122 of the end effector 120 is biased to the open state (or a fully opened state).

As discussed above, the trigger assembly 150 also includes a switch assembly 154. The switch assembly 154 illustratively includes a toggle switch 640, which is selectable to activate one or more switches 642. Activation of the switches 642 electrically energizes an electrical element 644, which electrically energizes the ultrasonic transducer 104 to engage the ultrasonic mode of the surgical instrument 102.

The elongated shaft assembly 112 includes an outer tubular sheath 650 and a rotation knob 652 coupled to the outer cylindrical sheath 650. The rotation knob 652 is operable to rotate the outer cylindrical sheath 650 about an axis defined by the outer cylindrical sheath 650. A reciprocating tubular actuator 654 is located within the outer tubular sheath 650 and mechanically engaged with the end effector 120 on a distal end. The reciprocating tubular actuator 654 is also mechanically engaged, on a proximal end, with the yoke 610 within the handle assembly 110 via coupling elements 656. In embodiments including an ultrasonic mode, an ultrasonic waveguide 670 is located within the reciprocating tubular actuator 654. A distal end of the ultrasonic waveguide 670 is acoustically coupled (e.g., directly or indirectly mechanically coupled) to the ultrasonic blade 130, and a proximal end is acoustically coupled to the transducer 104. The ultrasonic waveguide 670 may be isolated from other components of the elongated shaft assembly 112 by a protective sheath 672 and a number of isolation elements 674. The outer tubular sheath 650, the reciprocating tubular actuator 654, and the ultrasonic waveguide 670 are mechanically engaged together via a pin 658.

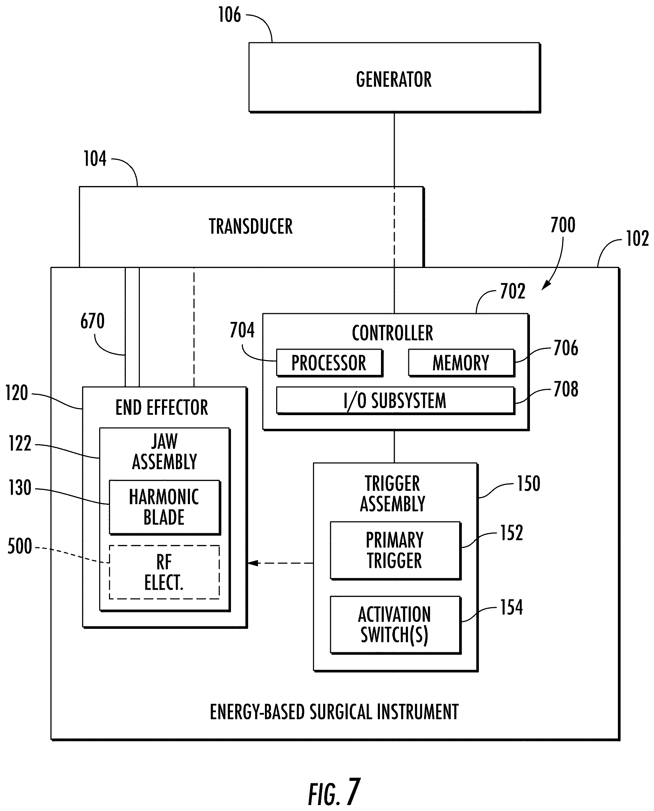

Referring now to FIG. 7, in the illustrative embodiment, the surgical instrument 102 includes a control circuit 700. The control circuit 700 includes a controller 702 and the trigger assembly 150, which cooperate to provide ultrasonic energy to the harmonic blade 130 of the jaw assembly 122 of the end effector 120 and/or RF energy to the RF electrodes 500 of the jaw assembly 122, depending on the operation modes of the surgical instrument 102 as discussed above. In other embodiments, however, the control circuit 700 may include additional or other electronic devices and/or circuit.

The controller 702 may be embodied as any type of controller, functional block, digital logic, or other component, device, circuitry, or collection thereof capable of performing the functions described herein. In illustrative embodiment, the controller 702 includes a processor 704, a memory 706, and an input/output (I/O) subsystem 708. The processor 704 may be embodied as any type of processor capable of performing the functions described herein. For example, the processor 704 may be embodied as a single or multi-core processor(s), digital signal processor, microcontroller, or other processor or processing/controlling circuit. Similarly, the memory 706 may be embodied as any type of volatile and/or non-volatile memory or data storage capable of performing the functions described herein. In operation, the memory 706 may store various data and software used during operation of the control circuit 700 such as executable firmware or software, programs, libraries, and drivers, which may be executed or otherwise used by the processor 704.

The processor 704 and memory 706 are communicatively coupled to other components of the control circuit 700 via the I/O subsystem 708, which may be embodied as circuitry and/or components to facilitate input/output operations between the controller 702 (e.g., the processor 704 and the memory 706) and the other components of the control circuit 700. For example, the I/O subsystem 708 may be embodied as, or otherwise include, memory controller hubs, input/output control hubs, firmware devices, communication links (i.e., point-to-point links, bus links, wires, cables, light guides, printed circuit board traces, etc.) and/or other components and subsystems to facilitate the input/output operations. In some embodiments, the I/O subsystem 708 may form a portion of a system-on-a-chip (SoC) and be incorporated, along with the processor 704 and the memory 706, and other components of the surgical instrument 102, on a single integrated circuit chip. Additionally, in some embodiments, the memory 706, or portions of the memory 706, may be incorporated into the processor 704.

During operation, as discussed above, the controller 702 is configured to control activation of an ultrasonic mode and/or an electro-surgical/RF mode of the surgical instrument 102. To do so, the controller 702 may monitor for activation of the primary trigger 152 and/or one or more activation switches 154 of the trigger assembly 150. In response to activation of the appropriate trigger 152 or switch 154, the controller 702 controls the transducer 104 to generate the ultrasonic energy, which is propagated to the harmonic blade 130 via the ultrasonic waveguide 670. Additionally or alternatively, in response to activation of a corresponding switch 154 of the trigger assembly 150, the controller 702 may be configured to supply an amount of RF energy, via the electro-surgical generator module 164 to the RF electrodes 500 via interconnections 710. It should be appreciated that, although the transducer 104 and the generator 106 are shown as separate components from the energy-based surgical instrument 102 in FIGS. 1 and 7, the transducer 104 and/or the generator 106 may be incorporated into the surgical instrument 102 in other embodiments.

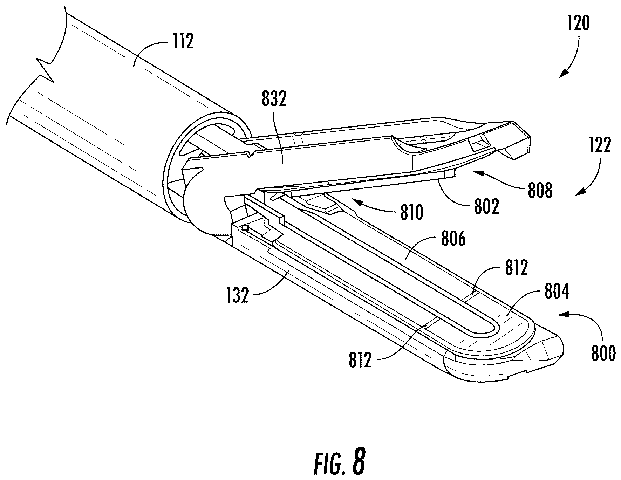

Referring now to FIG. 8, in another embodiment the jaw assembly 122 of the end effector 120 includes a pair of jaw clamps 132, 832, similar to those shown in FIG. 5B, above. In the illustrated end effector shown in FIG. 8, the jaw clamp 132 includes an electrode 800, and the jaw clamp 832 includes another electrode 802. In an embodiment, the electrode 800 may be embodied as an active electrode, and the electrode 802 may be embodied as a return electrode to facilitate the application of RF energy to tissue captured between the jaw clamps 132, 832. As shown, the electrode 800 includes a distal segment 804 and a proximal segment 806. Each of the segments 804, 806 may be independently energizeable in some embodiments. In some embodiments, the segments 804, 806 may be electrically insulated from each other. For example, in an embodiment the segments 804, 806 may be separated by a pair of insulated strips 812 as shown in FIG. 8.

Additionally or alternatively, in some embodiments the segments 804, 806 may be energizeable together. When wired together, insulated stops in the jaw assembly 122 may prevent conduction of RF energy between the proximal segment 806 and the return electrode 802 when the jaw assembly 122 is clamped closed without tissue between the jaw clamps 132, 832. Accordingly, in those embodiments, RF energy may be transferred through the distal segment 804, for example when at least a portion of the distal segment 804 is exposed through the jaw assembly 122 and a distal tip of the end effector 120 is pushed end-on against a bleeding source, such that energy flows from the distal segment 804 through the tissue to the return electrode 802.

Accordingly, the independently energizable distal-proximal segmented RF cutting electrode 800 (including distal segment 804 and proximal segment 806) may be used to perform distal-tip energized "nibbling" cutting and coagulation. In an embodiment, a bipolar electro-surgical (RF) instrument 102 may have a U-shaped coagulation electrode 800 on one jaw 132 and at least one return path electrode 802 on the opposing jaw 832. The distal tip of the electrode 800 may have a segmented distal portion 804 which is separately or cooperatively energizable with the main coagulation portion 806 of the electrode 800.

In some embodiments, the distal tip portion 804 may have a pressure-varying mechanism, which dependent on the jaw closure state may provide limited pressure (e.g., for traditional coagulation only) or concentrated pressure (e.g., tip nibbling/cutting). In an embodiment, the trigger for separate or cooperative activation of the electrode portions 804, 806 may be the sending or selection by the user of tissue only in the distal tip 804.

In some embodiments, a distal tip RF electrode shape and conformability may enable pressure in combination with RF energy to cause local distal tip tissue welding and bladeless cutting (i.e., "nibbling"). In some embodiments, the end effector 120 may include a rotational mechanism that creates a hump in either of the electrodes 800, 802. For example, a torsional blade mechanism as activated would rotate around its main axis to use the hump to increase distal tip 804 pressure. This increase in tip 804 pressure may enable lower displacement when firing RF energy.

In some embodiments, the jaws 132, 832 may close to differing final states based on whether the jaw had tissue enclosed and was operating in a coagulation mode, or only the tip had tissue enclosed and was nibbling-cutting. In an embodiment, the return electrode 802 may have a distal portion 808 and a proximal portion 810. In an embodiment, the distal bipolar electrode return path may have a spring deflectable portion, for example at the distal portion 808 of the electrode 802. The spring deflectable portion has a deflection stop limit. When the jaws 132, 832 are fully filled with tissue as for a coagulation mode, the return path 808 may deflect away, limiting the pressure applied at the distal tip. If tissue is only in the distal tip, the powered jaws 132, 832 could over-close, compressing the spring-biased tip 808 into its stop and beyond, thereby increasing the cutting-nibbling force or pressure.

In some embodiments, the distal deflectable tip may have a protrusion (i.e., a "mouse tooth") facing the tissue so that, when the stop is reached, the pressure density is very high in the center of the nibble and drops off moving away from the mouse tooth. This would manifest as a very high energy density when the stop is hit and the jaws 132, 832 are highly energized (meaning when the distal tip only is used, the power level could be different than when coagulation only is in operation). In some embodiments, the stop may be immovable or distally moveable. When the stop is distally moveable, the deflection stop may be pulled proximal to inhibit defection when nibbling is desired.

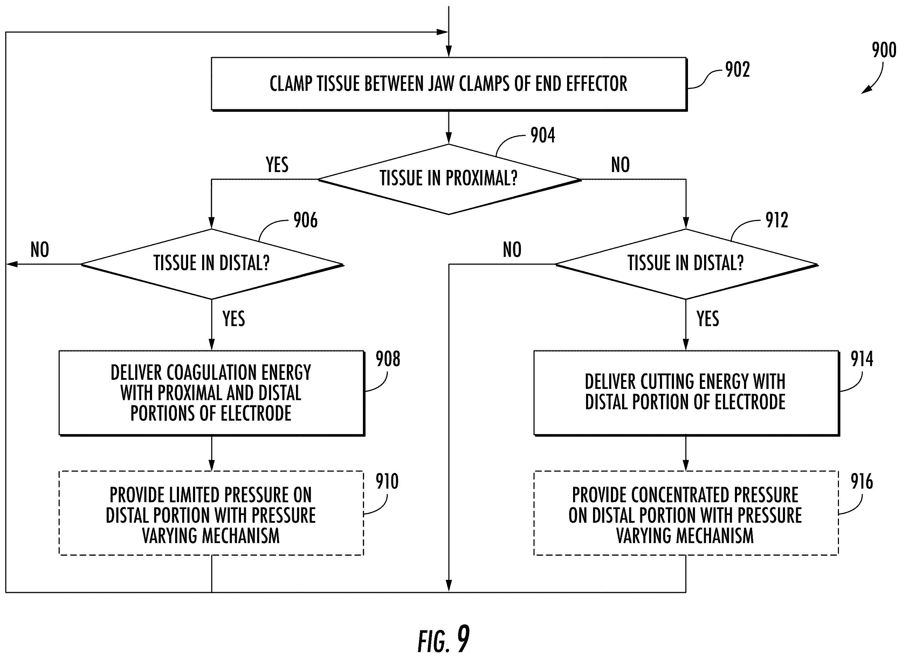

Referring now to FIG. 9, a method 900 for controlling an energy-based surgical instrument 102 is shown. The method 900 may be executed by the controller 702, the generator 106, and/or one or more other microcontrollers or other control elements of the system 100. The method 900 begins in block 902, in which tissue is clamped between jaw clamps of an end effector 120 of a surgical instrument 102 (e.g., the jaws 132, 832 of the end effector 120 shown in FIG. 8). The tissue may be clamped, for example when a user activates the trigger mechanism 152. In some embodiments, the jaw assembly 122 may be motorized, and the jaws may clamp the tissue in response to activation of a button or other input included in the switch assembly 154.

In block 904, the control element determines whether tissue is present within a proximal portion of the jaw assembly 122, for example located between the jaws 132, 832 at a location corresponding to the proximal segment 806 of the electrode 800. The presence of tissue in the proximal portion of the jaw assembly 122 may be determined by the final closure state of the jaw assembly 122 (e.g., final distance between the jaws 132, 832), by sensing the presence of tissue, for example by sensing impedance, or by any other technique. If tissue is present in the proximal portion of the jaw assembly, the method 900 branches to block 906, in which the control element determines whether tissue is present within a distal portion of the jaw assembly 122, for example located between the jaws 132, 832 at a location corresponding to the distal segment 804 of the electrode 800. The presence of tissue in the distal portion of the jaw assembly 122 may be determined using any appropriate technique as described above. If tissue is not present, the method 900 loops back to block 902 to continue performing control operations. If tissue is present within the distal portion of the jaw assembly 122 (and thus also within the proximal portion of the jaw assembly 122), the method 900 advances to block 908.

In block 908, the control element energizes the proximal segment 806 and the distal segment 804 cooperatively with a coagulation energy to perform a coagulation operation. In some embodiments, in block 910, limited pressure may be provided on the distal portion of the jaw assembly using a pressure varying mechanism, as described above. After delivering the coagulation energy, the method 900 loops back to block 902, to continue performing control operations.

Referring again to block 904, if tissue is not present in the proximal portion of the jaw assembly 122, the method 900 branches to block 912. In block 912, the control element determines whether tissue is present within the distal portion of the jaw assembly 122. The presence of tissue in the distal portion of the jaw assembly 122 may be determined using any appropriate technique as described above. If tissue is not present, the method 900 loops back to block 902 to continue performing control operations. If tissue is present within the distal portion of the jaw assembly 122 (and thus also not present within the proximal portion of the jaw assembly 122), the method 900 advances to block 914.

In block 914, the control element energizes the distal segment 804 independently from the proximal segment 806 with a cutting energy to perform a nibbling/cutting operation. As described above, the cutting energy is higher than the coagulation energy. In some embodiments, in block 916, concentrated pressure may be provided on the distal portion of the jaw assembly using a pressure varying mechanism, as described above. After delivering the cutting energy, the method 900 loops back to block 902, to continue performing control operations.

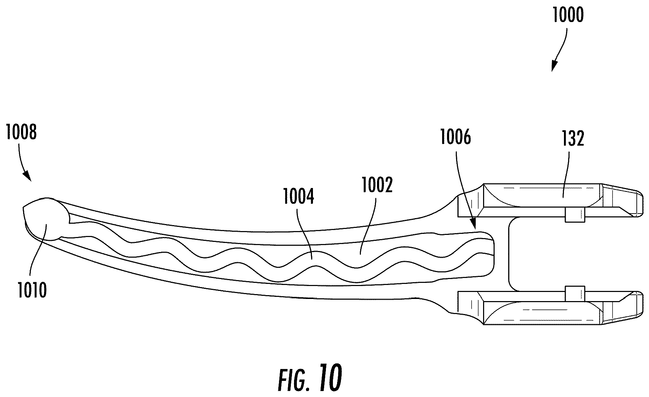

Referring now to FIG. 10, a jaw assembly 1000 includes a jaw clamp 132 similar to those in FIGS. 1-8 coupled to a tissue pad 1002. The tissue pad 1002 includes non-conductive portions, which may be formed from polytetrafluoroethylene (PTFE). The tissue pad 1002 is configured to receive an ultrasonic blade 130 when used in a combination ultrasonic/RF surgical instrument 102.

The jaw assembly 1000 further includes a compliant RF electrode 1004 positioned on an upper surface of the tissue pad 1002. The electrode 1004 extends from a proximal end 1006 to a distal tip 1008, and includes a distal tip feature 1010. The electrode 1004 is formed from conductive material, such as a metallic material, conductive ink, or another material. As shown, the electrode 1004 does not cover the entire surface of the non-conductive tissue pad 1002. Accordingly, the jaw assembly 1000 includes both non-conductive portions and conductive portions.

As shown, the jaw assembly 1000 may include an extension 1010 of the conductive portion of the RF compliant electrode 1004 to the distal surface of the clamp arm jaw 132, while maintaining distal retention of the electrode 1004 within the structural electrode support. The tip conductive RF electrode 1010 has a shape / geometry that enables distal tip RF nibbling / welding differently with a cambered clamp arm that causes distal tip contact with migration compression proximal.

In some embodiments, the electrode 1004 may be a conformal or erodible RF electrode 1004 for use with an ultrasonic blade 130. The distal tip 1010 of the electrode 1004 has a different retention shape or geometry than the proximal pad 1002 geometry, which may minimize unintended release of the distal portion of the electrode 1010 during high-temperature usage.

In some embodiments, the distal electrode geometry may have a differing track or pattern on the conductive portion 1004 in addition to the restrain portion 1002 which enables the distal tip to interact electrically at a bipolar tissue treatment before the proximal portion of the electrode has the conductive capability or interacting.

For example, a surgical instrument 102 with the jaw assembly 1000 may include tip geometry 1010 that may be used for end-on application of energy to bleeding sources. The non-conductive portion of the tissue pad 1002 may include geometry to hold the tip geometry 1010 in place. This geometry may be used with an ultrasonic blade 130, and may have geometry to minimize unintended release of the distal tip geometry 1010 during high temperature usage. In addition, when used with a deflectable electrode having deflection stops to prevent deflection from the ultrasonic blade 130, the jaw assembly may include additional stops or other features to retain the tip geometry 1010 and prevent shorting with the blade 130.

The following is a non-exhaustive list of embodiments which may or may not be claimed:
1. A method for controlling an energy-based surgical instrument, the method comprising:
   clamping tissue between a first jaw clamp and a second jaw clamp of an end effector of the energy-based surgical instrument, wherein the energy-based surgical instrument comprises a first electrode coupled to the first jaw clamp, the first electrode comprising a distal portion and a proximal portion, and wherein the energy-based surgical instrument further comprises a return electrode coupled to the second jaw clamp;
   independently energizing the distal portion to deliver radio frequency (RF) energy through the tissue between the first jaw clamp and the second jaw clamp to the return electrode when the tissue is only within the distal portion of the first jaw clamp; and
   cooperatively energizing the distal portion and the proximal portion to deliver RF energy through the tissue between the first jaw clamp and the second jaw clamp to the return electrode when the tissue is within the distal portion and the proximal portion of the first jaw clamp.
2. The method of embodiment 1, wherein:
   independently energizing the distal portion comprises delivering a cutting energy; and
   cooperatively energizing the distal portion and the proximal portion comprises delivering a coagulation energy.
3. The method of embodiment 2, wherein the cutting energy is higher than the coagulation energy.
4. The method of embodiment 1, further comprising:
   providing concentrated pressure on the distal portion of the first electrode with a pressure varying mechanism when independently energizing the distal portion; and
   providing limited pressure on the distal portion of the first electrode with the pressure varying mechanism when cooperatively energizing the distal portion and the proximal portion.

While the disclosure has been illustrated and described in detail in the drawings and foregoing description, such an illustration and description is to be considered as illustrative and not restrictive in character, it being understood that only illustrative embodiments have been shown and described and that all changes and modifications that come within the spirit of the disclosure are desired to be protected.

There are a plurality of advantages of the present disclosure arising from the various features of the methods, apparatuses, and systems described herein. It will be noted that alternative embodiments of the methods, apparatuses, and systems of the present disclosure may not include all of the features described yet still benefit from at least some of the advantages of such features. Those of ordinary skill in the art may readily devise their own implementations of the methods, apparatuses, and systems that incorporate one or more of the features of the present invention and fall within the spirit and scope of the present disclosure as defined by the appended claims.

## Claims

1. An energy-based surgical instrument comprising:
an end effector comprising a first jaw clamp and a second jaw clamp;
a first electrode coupled to the first jaw clamp, the first electrode comprising a distal portion and a proximal portion; and
a return electrode coupled to the second jaw clamp;
wherein the distal portion and the proximal portion are independently energizable to deliver radio frequency (RF) energy through tissue between the first jaw clamp and the second jaw clamp to the return electrode.

2. The surgical instrument of claim 1, wherein:
the proximal portion is configured to deliver a coagulation energy; and
the distal portion is configured to separately or cooperatively deliver the coagulation energy or a cutting energy.

3. The surgical instrument of claim 2, wherein the cutting energy is higher than the coagulation energy.

4. The surgical instrument of claim 2, wherein the distal portion is configured to separately deliver the cutting energy when tissue is only within the distal portion of the first jaw clamp.

5. The surgical instrument of claim 2, wherein the distal portion is configured to cooperatively deliver the coagulation energy or the cutting energy when tissue is within the distal portion and the proximal portion of the first jaw clamp.

6. The surgical instrument of any preceding claim, wherein the end effector further comprises a pressure varying mechanism that selectively provides limited pressure or concentrated pressure on the distal portion of the first electrode.

7. The surgical instrument of any preceding claim, wherein the end effector further comprises a rotational mechanism that rotates a hump under the first electrode or the second electrode, wherein when the hump is rotated, pressure on the distal portion of the first electrode is increased.

8. The surgical instrument of any preceding claim, wherein the return electrode comprises a distal portion, wherein the distal portion comprises a spring deflectable portion that is spring biased toward the first electrode and is deflectable away from the first electrode, preferably wherein the spring deflectable portion comprises a deflection stop limit that limits deflection of the return electrode away from the first electrode.

9. The surgical instrument of claim 8, wherein:
when the first jaw clamp and the second jaw clamp are closed with tissue between the distal portion and the proximal portion of the first electrode and the return electrode, the spring deflectable portion does not reach the deflection stop limit, and pressure applied at the distal portion is limited; and
when the first jaw clamp and the second jaw clamp are closed with tissue between the distal portion of the first electrode and the return electrode and without tissue between the proximal portion of the first electrode and the return electrode, the spring deflectable portion reaches the deflection stop limit, and increased pressure is applied at the distal portion.

10. The surgical instrument of claim 8 or claim 9, further comprising a protrusion extending from the distal portion of the return electrode toward the distal portion of the first electrode.

11. The surgical instrument of any one of claims 8 to 10, wherein the deflection stop limit is movable between a first position in which deflection of the return electrode is inhibited and a second position in which deflection of the return electrode is not inhibited.

12. An energy-based surgical instrument comprising:
an end effector comprising a jaw clamp, wherein the jaw clamp extends from a proximal end to a distal tip;
a tissue pad coupled to the jaw clamp and extending from the proximal end to the distal tip, wherein the tissue pad comprises a non-conductive top surface having proximal pad geometry; and
an electrode coupled to the top surface of the tissue pad and extending from the proximal end to the distal tip, wherein the electrode covers a first portion of the tissue pad and does not cover a second portion of the tissue pad, wherein the electrode comprises a distal feature that extends to the distal tip of the jaw clamp, and wherein the distal feature has a different retention shape or geometry than the proximal pad geometry.

13. The surgical instrument of claim 12, wherein the electrode comprises a conformal or erodible RF electrode for use with an ultrasonic blade.

14. The surgical instrument of claim 12 or claim 13, wherein the distal feature of the electrode interacts electrically with tissue before a proximal portion of the electrode interacts electrically with the tissue.

15. The surgical instrument of any one of claims 12 to 14, wherein the tissue pad comprises polytetrafluoroethylene (PTFE).
